# EUROPEAN PATENT SPECIFICATION

(11) **EP 0 370 107 B1**
(45) Date of publication and mention of the grant of the patent: **19.10.1994**
(21) Application number: 88906058.8
(22) Date of filing: 30.06.1988
(51) Int. Cl.: G10K 11/34

(54) **ULTRASONIC DIAGNOSTIC APPARATUS**
ULTRASCHALLDIAGNOSEGERÄT
APPAREIL DE DIAGNOSTIC ULTRASONIQUE

(30) Priority: 30.06.1987 JP 163227/87; 30.06.1987 JP 163228/87; 30.06.1987 JP 163229/87
(43) Date of publication of application: 30.05.1990
(73) Proprietor: YOKOGAWA MEDICAL SYSTEMS, LTD, Hino-shi, Tokyo 191 (JP)
(72) Inventor: SHIMAZAKI,Toru, Hino-shi,Tokyo 191 (JP); ANDO,Motoyoshi, Hino-shi,Tokyo 191 (JP); TABEI, Hiroshi, -chome Hino-shi Tokyo 190 (JP)
(74) Representative: Henkel, Feiler, Hänzel & Partner
(86) International application number: PCT/JP88/00660
(87) International publication number: WO 89/00026

(56) References cited:
- EP-A- 0 212 737
- JP-A- 5 611 374
- JP-A- 5 640 129
- JP-A-58 163 346
- US-A- 3 815 409
- US-A- 4 532 933

## Description

### TECHNICAL FIELD

The present invention is directed generally to an ultrasound diagnosing device adapted to transmit and receive convergent ultrasound beams, and more particularly, to an ultrasound diagnosing device arranged to uniformly converge a width of the beams in a direction orthogonal to a scanning direction of sound rays widely from a short range to a long range.

### BACKGROUND ARTS

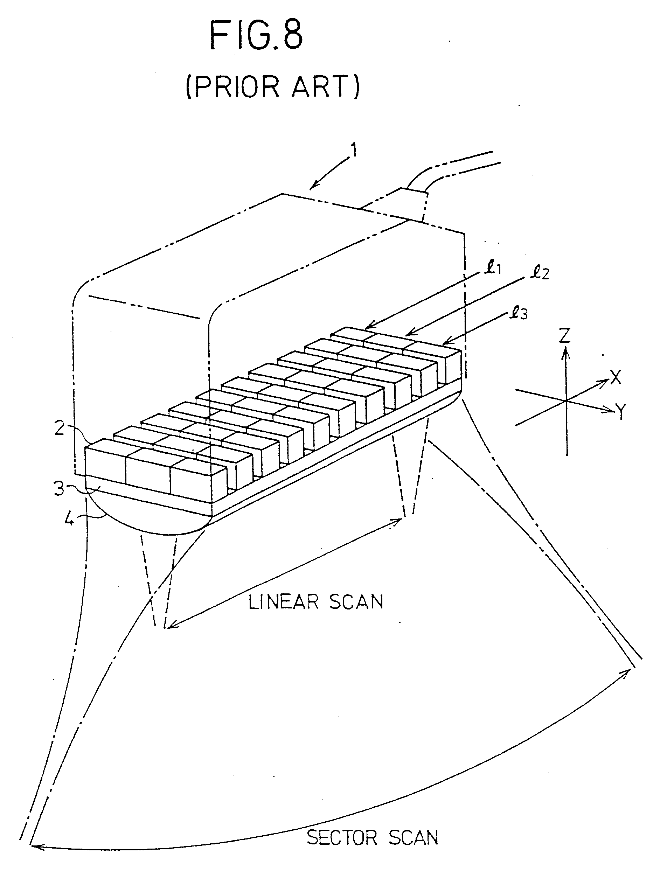

Disclosed in document JP-A-62-117539 is an example of a prior art ultrasonic diagnosing device in which to improve resolving power in a direction orthogonal to a scanning direction of sound rays. This type of ultrasound diagnosing device involves the use of an ultrasound probe having a configuration depicted in FIG. 8. Referring to FIG. 8, an ultrasound probe 1 generally designated at 1 is constructed of: an oscillator element group 2 arranged such that the elements are split at predetermined pitches in the sound ray scanning direction, i.e., a direction X, while in a direction y orthogonal to the sound ray scanning direction the elements are three-split to thereby constitute three arrays, viz., element arrays 11, 12 and 13 in the direction X; an acoustic matching layer 3 attached to an ultrasound radiation face thereof; and an acoustic lens 4 assuming a semi-circular shape. Note that a direction Z is vertical to the ultrasound radiation face. The thus constructed ultrasound probe performs, when a Y-directional aperture reaches its maximum in the case of a target being positioned deep, an X-directional scan, i.e., a sector scan indicated by, e.g., a one-dotted line by employing all the element trains 11, 12 and 13. During this scan, a Y-directional width of the ultrasound beams is converged by means of the acoustic lens 4. Whereas if the target is positioned shallowly, a linear scan is, as indicated by a broken line, effected by using the central oscillator element 12 alone. At this time, the Y-directional aperture is smaller than in the deep position, and the Y-directional width of the ultrasound beams is narrowed corresponding to the small aperture. In the ultrasound diagnosing device adaptive to vary the Y-directional aperture, Y-directional resolving power is improved from the shallow level to the deep level. In the prior art ultrasound diagnosing device, however, a focal point of the acoustic lens is set typically in a deep position, which in turn leads to such a problem that the ultrasound beams can not sufficiently be converged in the direction Y in the vicinity of the acoustic lens.

Videlicet, the problem is that it is impossible to obtain the resolving power which is uniform in the direction Y over a wide range from the vicinity of the acoustic lens down to a deep position.

Document JP-A-56 40 129 discloses an ultrasonic wave focusing apparatus which uses acoustic lenses, and document JP-A-56 11 374 describes an ultrasonic wave collecting apparatus having an electrode.

Further document US-A-4 532 933 discloses a composite lens having a two stage curvature configuration for a ultrasound probe, focusing into two deep and shallow regions. Finally, document EP-A-O 212 737 discloses a ultrasound probe having an electrode array along an X-Y plan, with central electrodes and side electrodes differently excited to scan in longitudinal and transverse directions.

### DISCLOSURE OF THE INVENTION

It is an object of the present invention to provide an ultrasound diagnosing device arranged to obtain the uniform resolving power in a direction orthogonal to a scanning direction of sound rays over a wide range from the shallow to the deep.

To solve this object the present invention provides an ultrasound diagnosing device as specified in claim 1.

In an ultrasound diagnosing device a plurality of unidimensional arrays of a plurality of ultrasound oscillators are arranged in parallel, and ultrasound signals are transmitted and received while changing combinations of the arrays associated with transmission and/or receiving of the ultrasound waves corresponding to a depth of an object for observation by use of an ultrasound probe mounted with an acoustic lens having an intrinsic focal distance for every array on the side of an ultrasound radiation face of those arrays.

### BRIEF DESCRIPTION OF THE DRAWINGS

Fig. 1 is a view of assistance in explaining a construction of fundamental components of an ultrasound probe for use with an ultrasound diagnosing device in an embodiment of the present invention;
FIG. 2 is a sectional view depicting the fundamental components of the ultrasound probe used for the ultrasound diagnosing device in the embodiment of the invention;
FIG. 3 is a block diagram illustrating an electric configuration of the ultrasound diagnosing device in the embodiment of the invention;
FIGS. 4A and 4B are diagrams each showing a state of ultrasound beams in the ultrasound diagnosing device of the invention;
FIG. 5 is a chart showing the states of the ultrasound beams in the ultrasound diagnosing device thereof;
FIGS. 6 and 7 are sectional views illustrating the fundamental components of the ultrasound probe employed in other embodiments of the invention; and
FIG. 8 is a view showing an example of the prior art.

### BEST MODE FOR CARRYING OUT THE INVENTION

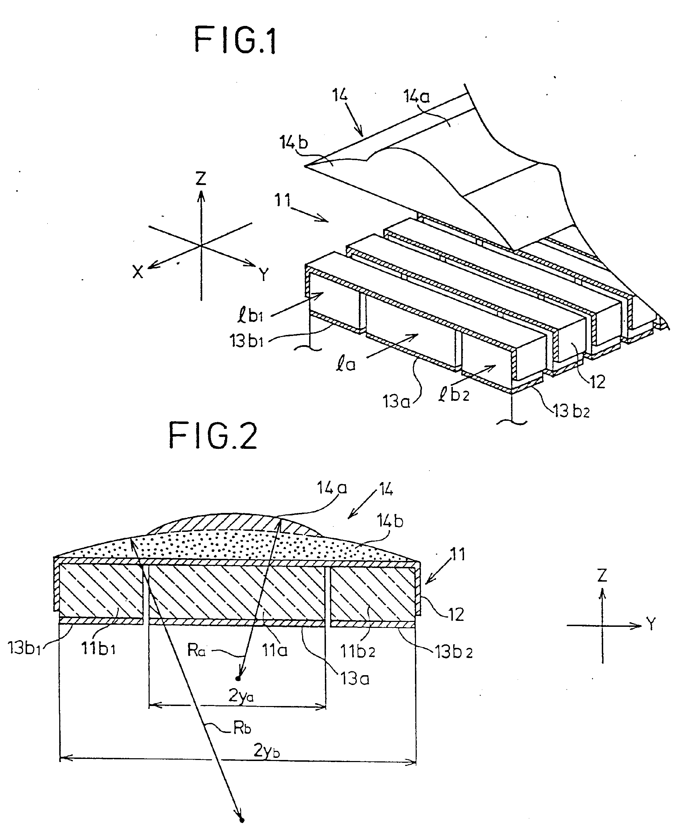

The description will start with a construction of an ultrasound probe employed in an embodiment of the present invention. Turning first to FIGS. 1 and 2, an ultrasound oscillator generally designated at 11 includes a rectangular piezoelectric body, a major axis, a minor axis and a thickness of which are respectively defined in directions X, Y and Z. The piezoelectric body is split in the direction X to form a plurality of oscillator elements at predetermined pitches and also in the direction Y to form three oscillator elements having a length ratio of 1 : 2 : 1. Such divisions provide X-directional three arrays consisting of a central element train la and two side element trains lb₁ and lb₂. Fixed to upper surfaces of the respective elements are common electrodes (first electrodes) 12 for electrically connecting the elements by threes in the direction Y, one ends of which are connected to a reference potential point of a circuit. Signal electrodes (second electrodes) 13a, 13b₁ and 13b₂ are fixed individually to lower surfaces of the elements. An acoustic lens 14 is made of rubber members. The acoustic lens 14 is composed of a sub-lens 14a having a curvature radius Ra and another sub-lens 14b having a curvature radius Rb ( > Ra), thus assuming a substantially semi-cylindrical configuration having the two stage curvatures. The sub-lens 14a forming a focal point Fa serves to cover the signal electrodes 13a, i.e., a Y-directional aperture of the central element train la. The sub-lens 14b forming a focal point Fb serves to cover the signal electrodes 13a, 13b₁ and 13b₂, viz., the Y-directional aperture defined by all the element trains la, lb₁ and lb₂. The thus constructed acoustic lens 14 is bonded to an ultrasound radiation face of the ultrasound oscillator 11. An acoustic matching layer is, if necessary, interposed between the acoustic lens 14 and the ultrasound oscillator 11. A backing member (not illustrated) is properly attached to a portion opposite to the ultrasound radiation face of the ultrasound oscillator 11.

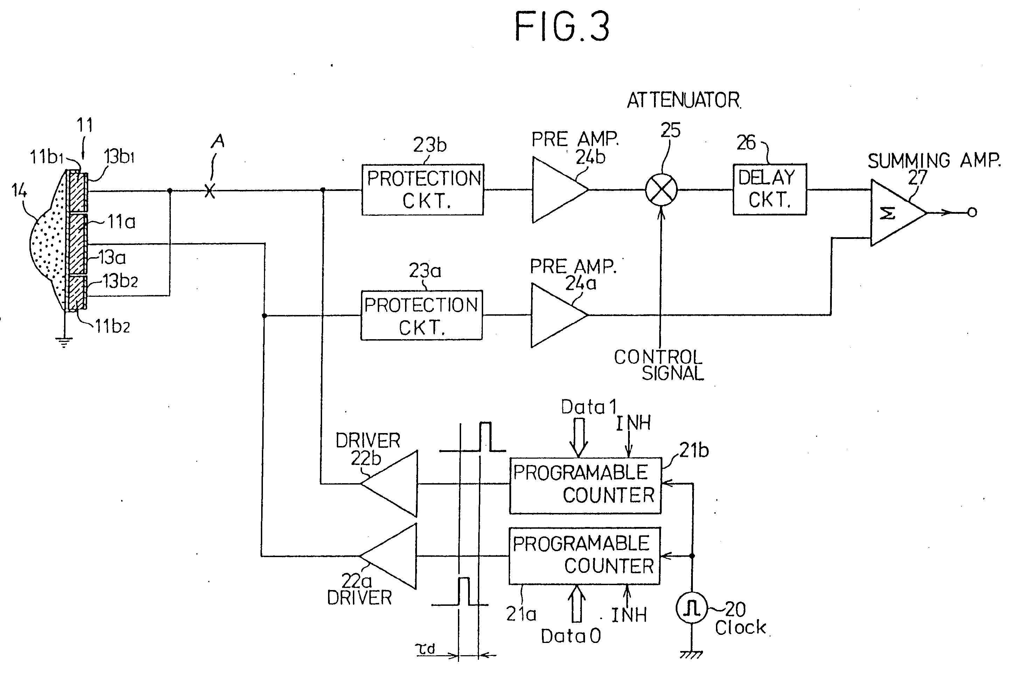

Referring to FIG. 3, there is illustrated a wave transmitting/receiving module with respect to one channel in the direction X, the module being connected to each element of the thus constituted ultrasound probe. The signal electrode 13a of the central element is connected to a first wave transmitting/receiving unit which will hereinafter be mentioned, while the signal electrodes 13b₁ and 13b₂ of the side elements are connected in common within the ultrasound probe and further connected to a second wave transmitting/receiving unit which will also be stated later. Because of the foregoing connection, a wave transmitting/receiving area associated with the signal electrodes 13a is equalized to a wave transmitting/receiving area associated with the signal electrodes 13b₁ and 13b₂. This in turn facilitates matching of driving circuits of the wave transmitting/receiving module connected to the respective signal electrodes. The first wave transmitting/receiving unit consists of a wave transmitting system including a programmable counter 21a and a wave transmitting driver 22a and of a wave receiving system including a protection circuit 23a and a preamplifier 24a. The second wave transmitting/receiving unit consists of a wave transmitting system including a programmable counter 21b and a wave transmitting driver 22b and of a wave receiving system including a protection circuit 23b, a preamplifier 24b, an attenuator 25 and a delay element 26. The attenuator 25 is so controlled by an unillustrated main control unit as to admit or hinder a passage of a receiving signal, and is also used for, as will be mentioned later, controlling the Y-directional aperture. Outputs of the two wave receiving systems are added by means of a summing amplifier 27, and the added value is imparted to a delay line (illustration is omitted) for forming the wave receiving beams preparatory to a phasing addition to wave receiving signals of other channels. Circuitry subsequent to the delay line for forming the receiving beams is common to an ordinary one in an electronic scan type ultrasound diagnosing device. The programmable counters 21a and 21b receive common clocks 20, and function on the basis of control signals IN and preset data which are imparted from an unillustrated main control unit, Under control of the main control unit, there is transmitted and received ultrasound waves either by the first wave transmitting/receiving unit or by a combination of the first and second wave transmitting/receiving units. The thus arranged wave transmitting/receiving units are provided for the respective signal electrodes arrayed in the direction X, viz., for all the channels. The X-directional ultrasound beams are scanned in the same manner as that in a known ultrasound diagnosing device. In this case, the aperture is changed in the direction Y depending on a depth of the object for observation.

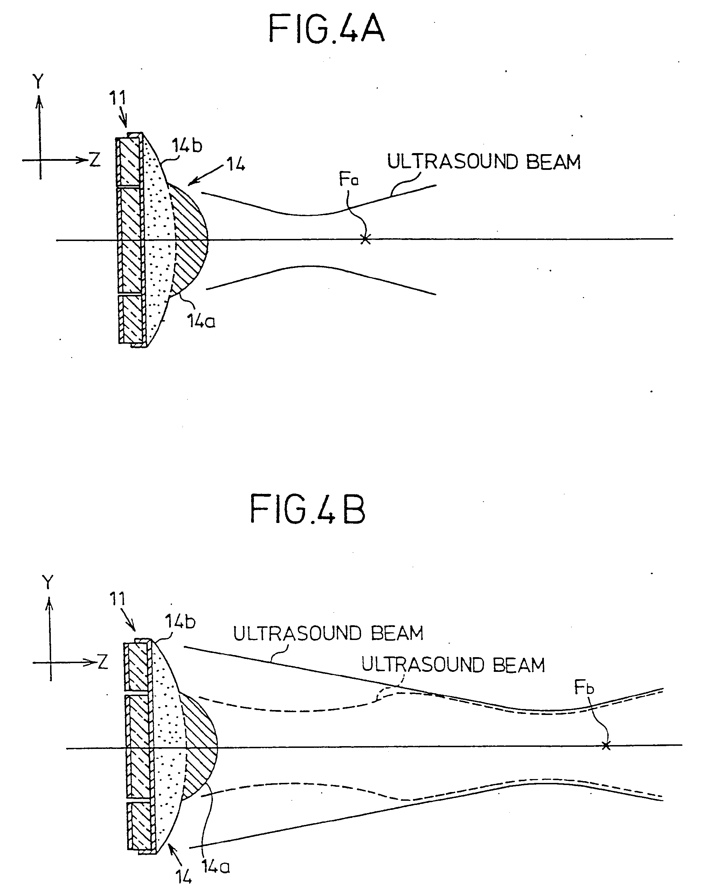

If a position of the objective part shallow, only the programmable counter 21a exclusive of the other counter functions under control of the main control unit, and gives forth output pulses by which the wave transmitting driver 22a is turned ON. Outputs of the driver 22a are applied to the signal electrodes 13a, thereby driving the elements 11a alone. The ultrasound waves generated by the elements 11a are changed into beams converged via the sub-lens 14a, and the object is irradiated with such convergent beams. Echoes from the objective part are detected by means of the elements 11a and then inputted via the protection circuit 23a to the preamplifier 24a, where the echoes are amplified. The signals amplified by the preamplifier 24a are outputted from the summing amplifier 27. The echo signals are detected also by the elements 11b₁ and 11b₂, but these detected signals are hindered by the attenuator 25 which remains hindered by the main control unit. The ultrasound beams transmitted and received at that time undergo a restriction by dimensions of the elements 11a in the central row, i.e., by a narrow aperture, and are converged via the sub-lens 14a having a small curvature radius Ra, i.e., a short focal distance Fa, with the result that the beams become narrow in the Y-directional width. FIG. 4A illustrates a configuration of the ultrasound beams transmitted at that time.

Next, whereas if deep, both of the programmable counters 21a and 21b work under the control of the main control unit. These programmable counters generate the output pulses at timings based on the respective preset data. The preset data are so prescribed that the output pulses of the programmable counter 21b are generated slower by a time-lag τd than the output pulses of the counter 21a. The wave transmitting driver 22b is turned ON slower by the time-lag τd than the driver 22a. Hence, the elements 11b₁ and 11b₂ are driven slower by the time-lag τd than the element 11a. The ultrasound waves generated by the elements 11a are changed into beams converged via the sub-lens 14a, while the ultrasound waves generated by the elements 11b₁ and 11b₂ are changed into beams converged via the sub-lens 14b. These convergent beams fall upon the objective part. The acoustic lens 14 has such a construction that the sub-lens 14a is superposed on the sub-lens 14b, whereby the ultrasound waves emitted from the sub-lens 14a are slower by a time equivalent to a thickness of a part of the sub-lens 14b on which the sub-lens 14a is disposed than the ultrasound waves emitted from the sub-lens 14b. The time-difference τd between the driving of the elements 11a and that of the elements 11b₁ and 11b₂ serves to compensate the delay of timing at which the ultrasound waves are emitted-i.e., it may be defined as a kind of electronic focus. Turning to FIG. 4B, there is depicted a configuration, drawn with a solid line, of the ultrasound beams transmitted. Echoes from the objective part which correspond thereto are detected by the elements 11a, 11b₁ and 11b₂. At an initial stage of receiving the echoes, as in the case of receiving the echoes from the foregoing shallow position, only the detection signals of the elements 11a are received in a state where the detection signals of the elements 11b₁ and 11b₂ are hindered by the attenuator 25. Therefore, at this stage the echoes are received by a small aperture, and the ultrasound waves received at that time come into beams converged via the sub-lens 14a. Subsequent to the stage of receiving the echoes from the shallow position, the attenuator 25 is changed to a signal passage state in which to receive the echoes with a full aperture. Namely, the signals detected by the elements 11a are amplified by the preamplifier 24a and then inputted to the summing amplifier 27. On the other hand, the signals detected by the elements 11b₁ and 11b₂ are amplified by the preamplifier 24b and thereafter imparted to the summing amplifier 27 via the attenuator 25 and the delay element 26. The delay element 26 behaves to delay the signals received by the elements 11b₁ and 11b₂ by a time τd, thereby compensating a time difference between the receiving signals which is caused due to a difference in thickness between the sub-lenses 14a and 14b-viz., this may be defined as an electronic focus with respect to receiving of the signals. The summing amplifier 27 acts to sum up the signals sent from the two wave receiving systems and output them to a main delay line. In this manner, the aperture for receiving the echoes is varied corresponding to the depth of the objective part. A configuration of the ultrasound beams received is illustrated with a broken line in FIG. 4B, wherein the width thereof is narrow over a wide range from the shallow location to the deep location.

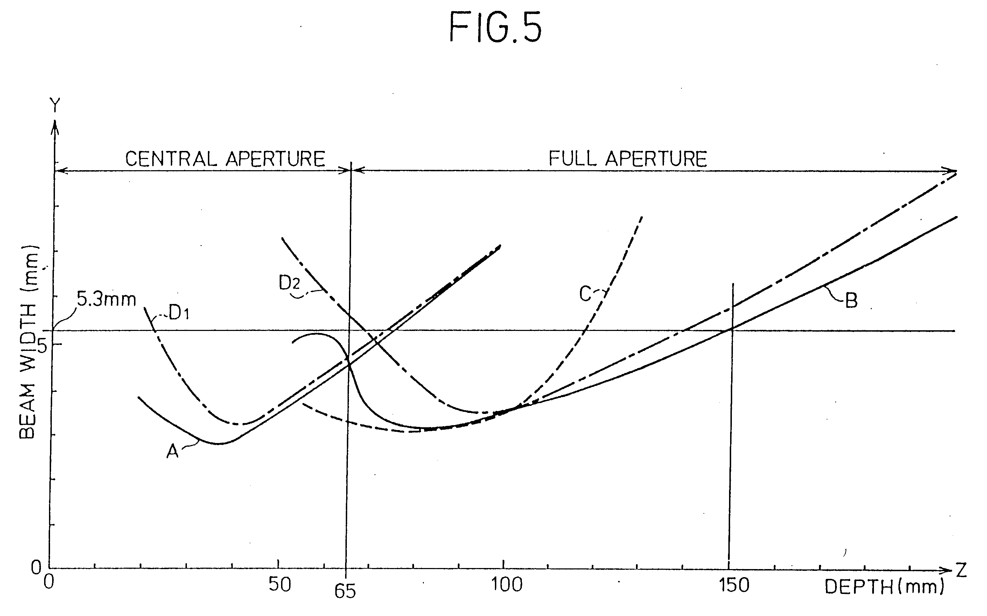

Referring to FIG. 5, there is given an example of characteristics of Y-directional resolving power in the ultrasound diagnosing device actually manufactured according to the present invention. The axis of abscissa of FIG. 5 indicates a depth (mm) of the ultrasound beam, while the axis of ordinate represents a width (mm) thereof. Note that detailed data on the acoustic lens employed are given as follows.
Sub-lens 14a : aperture 2ya = 7.5 mm, focal distance Fa = 50 mm
Sub-lens 14b : aperture 2yb = 15 mm, focal distance Fb = 100 mm
Acoustic lens corresponding-to-thickness time difference τd = 50ns.

A graph A shows a characteristic associated with the elements 11a in the central row, i.e., a central aperture characteristic. A graph B indicates a characteristic of the elements in all the rows, viz., a full-aperture characteristic with electronic focusing. A graph C shows a full-aperture characteristic with no electronic focusing. Graphs D1 and D2 exhibit characteristics when using an ultrasound probe having the same structure as that in the prior arts. More specifically, D1 shows a central aperture characteristic, and D2 indicates a full-aperture characteristic. Dimensions are the same as the above, The acoustic lens is classified as a single curvature lens having a focal point of 100 mm. Referring again to FIG. 5, a characteristic, which is developed when connecting the graph A to the graph B, is the one obtained on condition that the aperture is varied on the basis of a depth of 65 mm, and electronic focusing is effected in the case of the full-aperture. In accordance with this characteristic, a beam width (-6dB on one side) of 5.3 mm within a depth range of 20 - 150 mm is secured, It can be clarified that this characteristic, in which the Y-directional beam width is narrow over the entire depth range, has more superb resolving power than in the characteristic when connecting the graphs D1 and D2 to each other, i.e., when changing the aperture on the basis of a depth of approximately 70 mm by use of the ultrasound probe having the structure identical with that in the prior arts. In contrast, there is, it can be understood, produced more excellent resolving power inherent in the characteristic when connecting the graph A to the graph C, viz., the one when no electronic focusing is performed at the time of the full-aperture on condition that the aperture is varied on the basis of a depth of about 55 mm with a depth limit of 100 mm than in any cases given above. That is, according to the present invention, it is possible to improve the Y-directional resolving power over a wide range of depth for observation. If the observation depth is limited to a relatively shallow level, still higher resolving power can be acquired.

Note that the present invention is not limited to the embodiment discussed above. For instance, in FIG. 3, only the delay element 26 or a series circuit of the attenuator 25 and the delay element 26 is shifted to a spot on a common connecting line, indicated by a point A of the Figure, for the signal electrodes 13b₁ and 13b₂ within the ultrasound probe, and the output pulses of the driver 22a are supplied to the protection circuit 23b. With this arrangement, the electronic focusing and the aperture changing means or the electronic focusing means can be employed in common to the transmission and receiving. This in turn conduces to a reduction in costs by omitting one system of wave transmitting drivers.

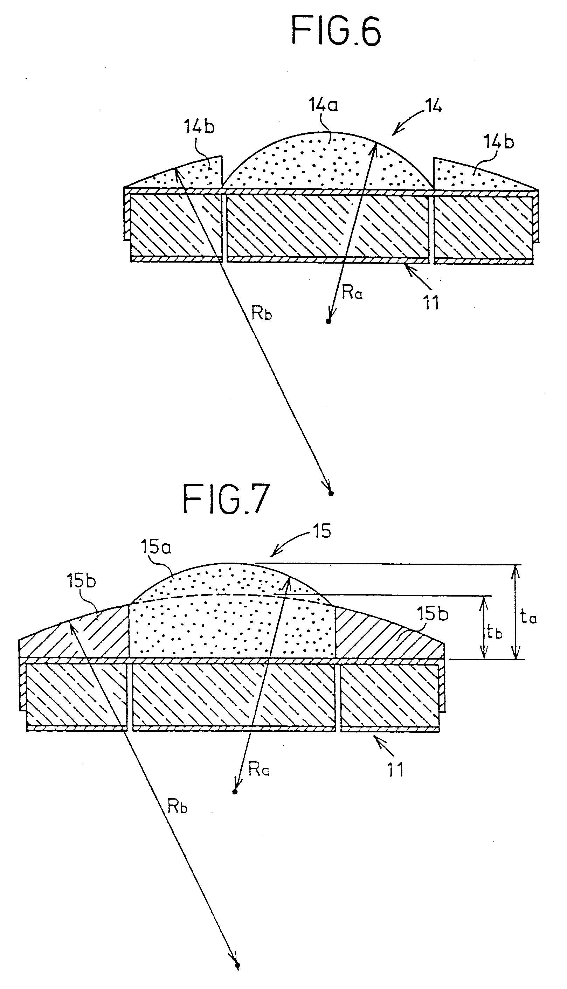

Note that in the above-described embodiment the acoustic lens 14 composed of rubber members assumes a substantially semi-cylindrical convex configuration having two stage curvatures. The member and configuration are not, however, limited to the above-mentioned. In short, when employing a member exhibiting a higher sound velocity than in traveling within a subject for examination, contrastingly it may suffice to use a concave lens having two stage curvatures. Besides, the number of stages of lens curvatures is not confined to two but may properly be selected. The acoustic lens may be formed in such a shape that, as depicted in FIG. 6, a raised-bottom of the sub-lens 14a is removed. As illustrated in FIG. 7, an acoustic lens having two stage curvatures is constructed by use of two kinds of rubber members 15a and 15b each having a different sound velocity. A time difference in the propagation delay between the two sub-lens may be eliminated by satisfying the following relationship.$\text{ta/tb = Ca/Cb}$
where ta and tb are the central thicknesses of the sub-lens having the respective curvatures, and Ca and Cb are the sound velocities. In the case of using the acoustic lenses depicted in FIGS. 6 and 7, it is unnecessary to provide, as described referring to FIG. 3, a time difference between the output pulses of the wave transmitting drivers of two systems, or provide the delay element 26. One practical arrangement in the ultrasound probe is that the common electrodes of the elements may be three-split in the direction Y. The ultrasound oscillators are not necessarily perfectly three-divided as shown in the embodiment. The ultrasound oscillators may include those functioning virtually as 3-divided ultrasound oscillators by selectively driving the 3-divided signal electrodes.

## Claims

1. An ultrasound diagnosing device mounted with an ultrasound probe driven by first electrodes (12) divided at predetermined pitches in a direction X and by second electrodes (13a, 13b1, 13b2) three-divided at a predetermined area ratio in a direction Y, said second electrodes (13a, 13b1, 13b2) being controlled to thereby vary a Y-directional aperture when transmitting and receiving waves in a direction Z orthogonal with said direction X and Y being defined as scan and slice directions,
characterized by comprising:
control means (25) for effecting a change to driving by said second electrodes (13a, 13b1, 13b2) in a central row or in all rows in accordance with a region concerned; an acoustic lens (14) assuming a substantially semi-cylindrical configuration (14b) and also a two stage curvature configuration (14a and 14b) to form a first focal point corresponding to an aperture by driving said second electrodes (13a, 13b1, 13b2) in the central row and a second focal point corresponding to an aperture by driving said electrodes in all the rows; and a delay element (26) provided on a signal line connected to said second electrodes (13a, 13b1, 13b2) in two side rows, wherein the waves are transmitted and received by said second electrodes (13a, 13b1, 13b2) in the central row in combination with a sub-lens (14a) having said first focal point with respect to a shallow location and by said second electrodes (13a, 13b1, 13b2) in all the rows in combination with a sub-lens (14b) having said second focal point with respect to a deep location, and a propagation time difference corresponding to a difference in thickness between said sub-lenses (14a) and (14b) is compensated by said delay element (26).

2. A device according to claim 1, characterized in that said second electrodes are three-divided at an area ratio of 1 : 2 : 1 in said direction Y.

## Patentansprüche

1. Ultraschall-Diagnosevorrichtung ausgerüstet mit einem Ultraschall-Meßfühler, der durch erste Elektroden (12), die an vorbestimmten Teilungen in einer X-Richtung geteilt sind, und durch zweite Elektroden (13a, 13b1, 13b2) betrieben wird, die in einem vorbestimmten Flächenverhältnis in einer Y-Richtung dreigeteilt sind, wobei die zweiten Elektroden (13a, 13b1, 13b2) gesteuert werden, um dadurch eine in Y-Richtung verlaufende Öffnung zu verändern, wenn Wellen in einer Richtung Z gesendet und empfangen werden, die zu den Richtungen X und Y orthogonal ist, die als Abtast- und Schnittrichtungen bestimmt sind,
dadurch gekennzeichnet,
daß enthalten sind:
Steuereinrichtungen (25) zum Bewirken einer Änderung des Betriebs durch die zweiten Elektroden (13a, 13b1, 13b2) in einer zentralen Reihe oder in allen Reihen entsprechend einer betroffenen Region, eine akustische Linse (14), die eine im wesentlichen halbzylindrische Anordnung (14b) und ferner eine zweistufige Krümmungsanordnung (14a und 14b) annimmt, um einen ersten Brennpunkt entsprechend einer Öffnung durch Betreiben der zweiten Elektroden (13a, 13b1, 13b2) in der zentralen Reihe und einen zweiten Brennpunkt entsprechend einer Öffnung durch Betreiben der Elektroden in allen Reihen zu bilden, und ein Verzögerungselement (26), das auf einer Signalleitung vorgesehen ist, die mit den zweiten Elektroden (13a, 13b1, 13b2) an zwei Seitenreihen verbunden ist, wobei die Wellen durch die zweiten Elektroden (13a, 13bl, 13b2) in der zentralen Reihe in Kombination mit einer Sub-Linse (14a), die den ersten Brennpunkt bezüglich einer nicht tiefen Stelle hat, und durch die zweiten Elektroden (13a, 13b1, 13b2) in allen Reihen in Kombination mit einer Sub-Linse (14b) gesendet und empfangen werden, die den zweiten Brennpunkt hinsichtlich einer tiefen Stelle hat, und eine Signallaufzeitdifferenz entsprechend einer Dickendifferenz zwischen den SubLinsen (14a) und (14b) durch das Verzögerungselement (26) kompensiert wird.

2. Vorrichtung nach Anspruch 1,
dadurch gekennzeichnet,
daß die zweiten Elektroden in einem Flächenverhältnis 1 : 2 : 1 in der Y-Richtung dreigeteilt sind.

## Revendications

1. Un appareil de diagnostic ultrasonique, sur lequel est montée une sonde ultrasonique commandée par des premières électrodes (12) divisées en des pas prédéterminés dans une direction X et par des secondes électrodes (13a, 13b1,13b2) divisées en trois suivant un rapport prédéterminé d'aire dans une direction Y, lesdites secondes électrodes (13a, 13b1, 13b2) étant commandées pour modifier ainsi une ouverture dans la direction Y lorsqu'elles émettent et reçoivent des ondes dans une direction Z orthogonale auxdites directions X et Y définies comme des directions de balayage et de tranche,
caractérisé en ce qu'il comporte :
des moyens de commande (25) pour effectuer une modification de la commande par lesdites secondes électrodes (13a, 13b1, 13b2) dans une rangée centrale ou dans toutes les rangées suivant une région concernée ; une lentille acoustique (14) présentant une configuration (14b) sensiblement semi-cylindrique et également une configuration (14a et 14b) de courbure à deux étages pour former un premier point focal correspondant à une ouverture par la commande desdites secondes électrodes (13a, 13b1, 13b2) dans la rangée centrale, et un second point focal correspondant à une ouverture par la commande desdites électrodes dans toutes les rangées ; et un élément à retard (26) prévu sur une ligne de signal reliée auxdites secondes électrodes (13a, 13b1, 13b2) dans deux rangées latérales, les ondes étant émises et reçues par lesdites secondes électrodes (13,13b1, 13b2) dans la rangée centrale en combinaison avec une sous-lentille (14a) présentant ledit premier point focal par rapport à un emplacement peu profond et par lesdites secondes électrodes (13a, 13b1, 13b2) dans toutes les rangées en combinaison avec une sous-lentille (14b) présentant ledit second point focal par rapport à un emplacement profond, et une différence de temps de propagation correspondant à une différence d'épaisseur entre lesdites sous-lentilles (14a) et (14b) étant compensée par ledit élément à retard (26).

2. Un appareil selon la revendication 1, caractérisé en ce que lesdites secondes électrodes sont divisées en trois suivant un rapport d'aire de 1 : 2 : 1 dans ladite direction Y.
